(19) Europäisches Patentamt
European Patent Office
Office européen des brevets

(11) EP 2 433 657 B1

(12) **EUROPEAN PATENT SPECIFICATION**

(45) Date of publication and mention of the grant of the patent:
**08.10.2014 Bulletin 2014/41**

(21) Application number: **09844998.6**

(22) Date of filing: **13.08.2009**

(51) Int Cl.:
***A61L 9/20*** $^{(2006.01)}$

(86) International application number:
**PCT/RU2009/000402**

(87) International publication number:
**WO 2010/134838 (25.11.2010 Gazette 2010/47)**

(54) **AIR STERILIZING ASSEMBLY**

LUFTSTERILISATIONSANORDNUNG

INSTALLATION DE STÉRILISATION DE L'AIR

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **20.05.2009 RU 2009118837**

(43) Date of publication of application:
**28.03.2012 Bulletin 2012/13**

(73) Proprietor: **YANEX INTELLECTUAL PROPERTY LIMITED**
**1066, Nicosia (CY)**

(72) Inventors:
- **Goldshtein, Yakov Abrammerovich**
**Moscow 115522 (RU)**
- **Shashkovsky, Sergei Gennadevich**
**Moscow 125319 (RU)**

(74) Representative: **Andrae | Westendorp Patentanwälte Partnerschaft**
**Uhlandstraße 2**
**80336 München (DE)**

(56) References cited:
**GB-A- 1 013 990**
**JP-A- 10 323 558**
**RU-A1- 94 018 873**
**RU-C1- 2 092 191**
**RU-C1- 2 092 191**
**US-A- 4 045 680**
**US-A- 4 503 360**
**US-A- 5 537 301**
**US-B1- 6 679 068**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 433 657 B1

Printed by Jouve, 75001 PARIS (FR)

## Description

Technical Field

The present invention relates to medical equipment, and can be used to improve the efficiency of disinfecting/sterilizing air in various rooms used for different purposes.

Prior art

Air disinfection/sterilizing devices with one or several sources of ultra-violet radiation in the form of mercury gas-discharge bactericidal lamps, and operating in a continuous lighting mode (RU 2153886 C1, A61L9/20, 2000; RU 2153886 C1, A61L9/20, 2007; EP 0220050, A61L9/18, 1987) are known from the prior art. The main disadvantage of these well-known devices is low productivity, caused by low intensity monochromatic radiation emitted from the used bactericidal lamps, and, accordingly, contributing to longer exposure periods needed to ensure effective air disinfection.

The another well-known unit used for air disinfection and deodorization, contains a body housing a power supply and control unit, which, in turn, includes an energy storage capacitor, a high-voltage constant current source, an ignition pulse generator, ferrite-core pulse transformer, and a control circuit, together with an ultra-violet source in the form of a pulsed gas-discharge lamp mounted on the body and enclosed in a tubular quartz casing, cooled by water; wherein the energy storage capacitor and the gas-discharge lamp form a discharge circuit, connected to the ignition pulse generator through the ferrite-core pulse transformer (RU 2092191 C1, A61L9/20, 1997). The water cooling system used in the pulsed gas-discharge lamp complicates design, increases the weight of construction, and reduces the efficiency of air disinfection processes.

Invention Disclosure

The present invention is aimed at simplifying the unit design and improving the efficiency and quality of air disinfection/sterilization.

The solution to this task is ensured by the following: given that the air disinfection device contains the body housing a power supply and control unit, which, in turn, includes an energy storage capacitor, a high-voltage constant current source, an ignition pulse generator, a ferrite-core pulse transformer, and a program control unit, together with an ultra-violet source in the form of a pulsed gas-discharge lamp, mounted on the body and enclosed in a tubular quartz casing, given that the energy storage capacitor and the pulsed gas-discharge lamp form a discharge circuit, connected to the ignition pulse generator through the ferrite-core pulse transformer, according to the invention, the pulsed gas-discharge lamp is placed in bactericidal radiation transparent casing, with an option of convective air-cooling due to natural draught inside the casing; in addition, there are one or several orifices of the upper level in the upper level and one or several orifices of the lower level in the lower level of the casing according to the following ratio of parameters:

$$h \cdot \frac{S^2_{upper} \cdot S^2_{lower}}{S^2_{upper} + S^2_{lower}} = \frac{1}{A} \cdot C^2 U^4_0 F^2 \qquad (1)$$

where h is the distance between the orifices/openings of the upper and lower levels, m;
$S_{upper}$ - total surface area of the orifices of the upper level, $m^2$;
$S_{lower}$ - total surface area of the orifices of the lower level, $m^2$;
$A = (2 \div 30) \cdot 10^{13}$ $J^2/m^5\ s^2$ - power correlation coefficient;
$C$ - capacity of energy storage capacitor, F;
$U_0$ - charge voltage of the energy storage capacitor, V;
$F$ - pulse repetition rate of the ignition pulse generator, Hz.

It is preferable for the ultra-violet radiation source to be installed vertically on the body.

The pulsed gas-discharge lamp can be U-shaped or cylindrical.

Furthermore, the orifices of the lower level can be made on the lateral surface of the casing.

In addition, the ultra-violet radiation source can be mounted horizontally, and the orifices of both the upper and lower levels can be made on the lateral surface of the casing.

Providing a casing with orifices of the upper and lower levels makes convective cooling of the pulsed gas-discharge lamp through ascending air flow, which passes through the casing as natural draught. This will simplify the design of the source of ultra-violet radiation and the unit as a whole, whereas the fore-mentioned ratio (1), which includes

the power correlation coefficient and associated structural and power (operating) parameters of the unit, and which is achieved through experimentation, ensures that A = $(2 \div 30)\cdot 10^{13}$ $J^2/m^5$ $s^2$ will provide, in the fore-mentioned range, both optimal and efficient simultaneously flowing processes of natural convection and cooling of the pulsed gas-discharge lamp, and processes of ozone formation from oxygen in the air thanks to short-wave ultra-violet radiation and thermal disintegration of ozone due to heat emitted through pulsed electric discharges in the gas-discharge lamp. This then enables the pulsed gas-discharge lamp to function reliably, and ensures a high degree of air disinfection, and a low level of ozone production in the processed premises.

Brief Description of the Drawings

Fig. 1 represents a general view of the air disinfection device with a vertical U-shaped gas-discharge lamp.
Fig. 2 represents the same device, but with a vertical cylindrical gas-discharge lamp.
Fig. 3 represents the design of a pulsed gas-discharge lamp in a horizontal arrangement.

Variants of Invention Implementation

The air disinfection device contains a body 1, housing a power supply and control unit, which, in turn, includes an energy storage capacitor 2, connected to a high-voltage constant current source 3, an ignition pulse generator 4, which is connected to a ferrite-core pulse transformer 5, and a program control unit 6. The source of ultra-violet radiation in the form of a pulsed gas-discharge lamp 7, comprising an energy storage capacitor 2, and a secondary winding of the ferrite-core pulse transformer 5 in a closed discharge circuit, is mounted on the body 1.

The pulsed gas-discharge lamp 7 in the form of a quartz U-shaped tube (Fig. 1) or in a cylindrical form (Fig. 2), having a cavity filled with inert Xenon gas at a pressure measuring 300 ÷ 450 Torr, and welded electrodes 8 at the end poles, made from thoriated tungsten, is enclosed in a bactericidal radiation transparent casing 9 (for example, made from fused quartz, whose spectral transparency range usually varies from 185 ÷ 2,700 nm, or made from sapphire). This lamp has orifices 10 of the lower level and orifices 11 of the upper level, the distance between them equaling "*h* ". The casing 9 and the pulsed gas-discharge lamp 7 are mounted preferably in a vertical position on the body 1 by means of a dielectric flange 12.

If the pulsed gas-discharge lamp 7 has a straight cylinder shape (Fig. 2), the orifice 11 of the upper level and the orifice 10 of the lower level can be made on the lateral surface of the cylindrical casing 9, the distance between the 10 and 11 orifices equaling "$h_1$". It is also possible to place the lower-level orifice 13 in the lower dielectric flange 12, and upper-level orifice 14 in the upper dielectric flange 15, the distance between orifices 13 and 14 equaling "$h_2$".

In another embodiment, pulsed gas-discharge lamp 7 horizontally fixed on the casing 9 (Fig. 3) has a lower-level orifice 10, which can be made in the middle of the lower part of the lateral surface of the casing 9, and the upper-level orifice 11 can be made in the upper part of the lateral surface of the casing 9, closer to the end poles, the distance between orifices 10 and 11 equaling "$h_3$", and/or in the dielectric flange 16 (orifices 14), the distance between orifices 10 and 14 equaling "$h_4$".

The air disinfection device is featured by experimental ratios (1), associating designed geometrical parameters of the casing 9 and power (operational) parameters for using the pulsed gas-discharge lamp 7:

$$h\cdot \frac{S^2_{upper}\cdot S^2_{lower}}{S^2_{upper}+S^2_{lower}} = \frac{1}{A}\cdot C^2U^4_0F^2 \qquad (1)$$

where h is the distance between the orifices of the upper and lower levels, m;
$S_{upper}$ - total surface area of the orifice in the upper level, $m^2$;
$S_{lower}$ - total surface area of the orifice in the lower level, $m^2$;
$A = (2 \div 30)\cdot 10^{13}$ $J^2/m^5$ $s^2$ -power correlation coefficient;
*C* - capacity of energy storage capacitor, F;
$U_0$ - charge voltage of the energy storage capacitor, V;
*F* - pulse repetition rate of the ignition pulse generator, Hz.

The fore-mentioned range $A = (2 \div 30)\cdot 10^{13}$ $J^2/m^5$ $s^2$ provides for both optimal and efficient simultaneously flowing processes of natural convection in the casing 9, and cooling of the pulsed gas-discharge lamp 7, and processes of ozone formation from oxygen in the air due to short-wave ultra-violet radiation and thermal disintegration of ozone

due to heat emitted through electric pulse discharges in the gas-discharge lamp 7. This enables the pulsed gas-discharge lamp 7 to function reliably, and ensures a high degree of air disinfection/sterilization, and a low level of ozone production in the processed premises.

Beforehand, calibrated results of microbiological tests are entered into the program control unit 6. These tests define irradiation periods required in specific spaces of the premises to attain required bactericidal efficiency and good quality air disinfection in order to assign particular technical parameters, corresponding to the ratio (1).

The air disinfection device operates in the following manner:

Before actual work operations are initiated, the device is installed in the middle of the room; the operator proceeds to enter data about the space (volume) in the room and required disinfection levels into the program control unit 6. Then, the operator switches on the unit, and leaves the room.

After a set delay time (20 to 30 seconds), the program control unit 6 switches on the constant current high-voltage source 3, which charges the energy storage capacitor 2. On reaching the required voltage (basically, $U_0$ = 1.4 ÷ 2.8 kV), the control circuit 6 switches off the constant current source 3, and starts the ignition pulse generator 4, which produces impulses of 0.7 to 1.5 kV voltage and 0.1 to 1 μs duration, generating a corresponding current in the primary winding of the pulse transformer 5. Because the two windings are electromagnetically coupled through the common ferrite core, impulse voltages up to 20 kW are induced in the secondary winding of the pulse transformer 5. This voltage is applied across electrodes 8 and causes primary electric breakdown of Xenon gas in the cavity of the pulsed gas-discharge lamp 7. The energy storage capacitor 2 discharges through the conductive plasma channel. Then, this primary plasma is strongly ionized, heated up, and expands, filling up all the internal space of the cavity of the pulsed gas-discharge lamp 7. Plasma temperature reaches 12 to 18 kK at the peak of the discharge current pulse. Such optically dense plasma emits a strong ultra-violet and visible radiation with continuous spectrum completely covering the bactericidal radiation range (205 to 305 nm). This radiation passes through the bactericidal radiation transparent tube of the pulsed gas-discharge lamp 7 and the casing 9, reaches the surrounding air space of the premises, and proceeds to disinfect the required areas.

After the energy storage capacitor 2 is discharged, the current stops flowing through the plasma; the plasma cools down, becomes deionized, and returns to its usual molecular state. Next, the process is repeated at the rate defined by the power of the high-voltage constant current source 3 (basically, $F$ = 2 to 4 Hz). The device is switched off when the determined time for the processing has been terminated.

Discharge current impulses and, correspondingly, radiation impulses are repeated as the periodic sequence. Half-amplitude width of the impulse current is 80 to 120 μsec, whereas the impulse repetition period ranges from 300 to 500 msec, that is, the energy reserved in the energy storage capacitor is injected into the plasma for a very short time, namely 3,000 to 5,000 times less than the inter impulse time.

Each ultra-violet radiation impulse, more exactly, the ultra-violet radiation with a wavelength less than 210 nm, causes forming some air-derived ozone in the space between the casing 9 and the pulsed gas-discharge lamp 7. This ozone cannot make its way immediately into the surrounding environment, as it is held back by casing 9, and then requires a considerable amount of time to exit spontaneously through the orifices in the casing.

As the device continues to work, the air-ozone mix in the space between casing 9 and the pulsed gas-discharge lamp 7 heats up due to substantially high thermal emissions from the tubular surface of the pulsed gas-discharge lamp 7 (about 40 % of the input power is transformed into heat in these applied modes), and its temperature continues to increase rapidly. Experimental data shows that the wall temperature of the casing 9, the tube of the pulsed gas-discharge lamp 7, and the gas (ozone-air mix) in the open space between these parts reaches a quasi-stationary value even after 40 to 80 second from the actual beginning of working.

Heating up the ozone-air mix in the space between the casing 9 and the tube of the pulsed gas-discharge lamp 7 activates two simultaneous processes: first, at a high temperature (more than 200 °C), ozone quickly decomposes into oxygen atoms, and immediately adopts a molecular form, and second, due to reduced gas density (ozone-air mix) when this mix is warmed up, there is a considerable difference between the density and the pressure inside and outside the casing 9. This causes an ascending convective air (ozone-air mix) stream to appear inside the casing from the lower level orifices to the upper level orifices (natural draught effect). This ascending flow of warm air, where ozone has already been restored to oxygen molecules, carries away all excess heat, cools the pulsed gas-discharge lamp 7 through natural convection, and exits the casing 9 practically free of ozone.

The interrelation between the indicated processes is reflected in the fore-mentioned ratio (1) between the geometrical parameters of the casing 9 and operating modes of the pulsed gas-discharge lamp 7. The experimentally defined power correlation coefficient A can be of values from $2 \cdot 10^{13}$ to $3 \cdot 10^{14}$ $J^2/m^5s^2$. Therefore, the following are established and provided for within this range: optimum balance between intensive emission of ultra-violet and visible radiation from the ultra-violet radiation source, and generation of a considerable amount of heat in the ozone-air mix on the one hand, and the convective cooling of the tubular surface of the pulsed gas-discharge lamp 7 by the air flow caused

by the natural draught on the other hand. In this way, the lower value of factor A corresponds to attaining high temperatures of the ozone intensive pyrolytic decomposition, whereas the higher value corresponds temperature limits for the lamp bulb walls softening in the pulsed gas-discharge lamp owing to excess heating.

Increasing of the power supplied to the pulsed gas-discharge lamp 7 causes the more ultra-violet radiation to be produced. This in turn results in increasing of ozone generation inside the source of ultra-violet radiation, in increasing of heat generation, in increasing of the temperature of the ozone-air mix, in increasing of the speed of the ozone thermal decomposition, and in increasing of the flow rate of air through the orifices in the casing 9, and at last in the better conditions for the convective cooling in the pulsed gas-discharge lamp 7.

Example 1, not according to the invention

The 1 kW air disinfection device, with a U-shaped pulsed gas-discharge lamp, positioned vertically (Fig. 1) in a quartz casing, 50 mm in diameter, has the following technical parameters:

$$C = 100\ \mu F = 10^{-4}\ F,\ U_0 = 2{,}800\ V,\ F = 2.5\ Hz,\ h = 0.275\ m,\ S_{upper} = 2 \cdot 10^{-4}\ m^2,$$

$$S_{lower} = 1.7 \cdot 10^{-4}\ m^2.$$

The device is installed in the room of following dimensions: length: 3.70 m, width: 3.05 meters, height: 3.85 m.; total surface area of the room 29.2 $m^3$. Previously conducted microbiological tests with the unit of these parameters showed that the unit should work for 36 seconds in order to maintain efficient bactericidal air disinfection at 99.9 % (i.e. 999 from every 1,0000 microorganisms are destroyed), measured for the *Staphylococcus Aureus* sanitary-indicative microorganism. The device was switched on by means of the program control unit 6 at the designated time, then the ozone level in the air was determined by the GANK-4 (gas analyzer), the concentration of ozone in the air and the average volume concentration of ozone in the air were calculated. The latter was equal to 49 $\mu g/m^3$.

This value does not exceed MAC of ozone in the air in compliance with Sanitary-hygienic standard GN 2.2.5.1313-03 "Maximum Allowable Concentration (MAC) of harmful substances permitted in the air in working zones" - 100 $\mu g/m^3$.

Example 2, not according to the invention.

The air disinfection device having power of 200 W, with a pulsed gas-discharge lamp in the form of a straight cylinder, positioned horizontally (see Fig. 3) in a quartz casing, 20 mm in diameter, has the following technical parameters:

$$C = 60\ \mu F = 6 \cdot 10^{-5}\ F,\ U_0 = 1{,}400\ V,\ F = 3.3\ Hz,\ h = 20\ mm = 0.02\ m,\ S_{upper} = 1\ cm^2 = 1 \cdot 10^{-4}\ m^2,\ S_{lower} = 1\ cm^2 = 1 \cdot 10^{-4}\ m^2.$$

The device is installed in the same room with a total surface area of 29.2 $m^3$. According to microbiological tests to maintain efficient bactericidal air disinfection at 99.9 %, measured for the *Staphylococcus Aureus* sanitary-indicative microorganism, this unit should work for 300 seconds to achieve maximum results. The device was switched on by means of the program control unit 6 at the designated time, then, the average volume of ozone concentration in the air was calculated, which, in this case, was equal 84 $\mu g/m^3$. The received value should also not exceed MAC of ozone permitted in the air in working zones.

## Claims

1. Air disinfection device comprises a body that houses the power supply and control unit comprising a storage capacitor, a high-voltage DC power supply, an ignition pulse generator, a ferrite-core pulse transformer and a program control unit, and an ultraviolet radiation source mounted on the body in the form of a pulse gas discharge lamp enclosed in a tubular cooled casing transparent to bactericidal radiation, wherein the storage capacitor and the pulse gas discharge lamp form a discharge circuit connected to the ignition pulse generator through the ferrite-core pulse

transformer, **characterized in that** the pulse gas discharge lamp is installed in a casing transparent to bactericidal radiation with convection air cooling capabilities provided by natural draft produced inside the casing, wherein the casing has one or several upper-level orifices in its upper part and some lower-level orifices in its lower part, with the following ratio of parameters:

$$h \cdot \frac{S^2_{upper} \cdot S^2_{lower}}{S^2_{upper} + S^2_{lower}} = \frac{1}{A} \cdot C^2 U^4_0 F^2 \quad (1)$$

where h is the distance between the orifices of the upper and lower levels, m;
$S_{upper}$ - total surface area of the orifice in the upper level, $m^2$;
$S_{lower}$ - total surface area of the orifice in the lower level, $m^2$;
$A = (2 \div 30) \cdot 10^{13}\ J^2/m^5\ s^2$ - power correlation coefficient;
$C$ - capacity of energy storage capacitor, F;
$U_0$ - charge voltage of the energy storage capacitor, V;
$F$ - pulse repetition rate of the ignition pulse generator, Hz.

**2.** Device according to Claim 1, **characterized in that** the ultraviolet radiation source is installed vertically on the body.

**3.** Device according to Claim 2, **characterized in that** the pulse gas discharge lamp is U-shaped.

**4.** Device according to Claim 1 or 2, **characterized in that** the lower-level orifices are made in the side surface of the casing.

**5.** Device according to Claim 1, **characterized in that** the ultraviolet radiation source is installed horizontally, and the upper- and lower-level orifices are made in the side surface of the casing.

## Patentansprüche

**1.** Luftentkeimungsanlage mit einem Gehäuse mit einer darin befindlichen Stromversorgungs- und Steuerungseinheit mit einem Speicherkondensator, einer Hochspannungsgleichstromquelle, einem Zündimpulsgeber, einem Pulstransformator mit Ferritkern und einer Programmsteuerungseinheit, sowie einer am Gehäuse befestigten UV-Strahlungsquelle in Form einer Puls-Gasentladungsröhre, die in einer rohrförmigen, für eine keimtötende Strahlung transparenten und gekühlten Ummantelung eingeschlossen ist, wobei der Speicherkondensator und die Puls-Gasentladungsröhre einen Entladestromkreis bilden, der mit dem Zündimpulsgeber über den Pulstransformator mit Ferritkern verbunden ist, **dadurch gekennzeichnet, dass** die Puls-Gasentladungsröhre in einer für die keimtötende Strahlung transparenten Ummantelung mit einer Konvektionsluftkühlung aufgrund der Erzeugung eines natürlichen Luftzuges im Innern der Ummantelung angeordnet ist, wobei im oberen Teil der Ummantelung eine bzw. mehrere Öffnungen einer oberen Ebene und im unteren Teil der Ummantelung Öffnungen einer unteren Ebene ausgeführt sind, mit folgender Beziehung von Parametern:

$$h \cdot \frac{S^2_{ober} \cdot S^2_{unter}}{S^2_{ober} + S^2_{unter}} = \frac{1}{A} \cdot C^2 U^4_0 F^2$$

wobei:

h = der Abstand zwischen den Öffnungen der oberen und der unteren Ebene, m;
$S_{ober}$ = die Gesamtfläche der Öffnungen der oberen Ebene, $m^2$;
$S_{unter}$= die Gesamtfläche der Öffnungen der unteren Ebene, $m^2$;
$A = (2 \div 30) \cdot 10^{13} J^2 (m^5 s^2)$ - energetischer Anpassungskoeffizient;
C = die Kapazität des Speicherkondensators, F;
$U_n$ = die Ladespannung des Speicherkondensators, V;

F = die Pulsfrequenz des Zündimpulsgebers, Hz.

**2.** Anlage gemäß Anspruch 1, **dadurch gekennzeichnet, dass** die UV-Strahlungsquelle am Gehäuse senkrecht angebracht ist.

**3.** Anlage gemäß Anspruch 2, **dadurch gekennzeichnet, dass** die Puls-Gasentladungsröhre U-förmig ausgeführt ist.

**4.** Anlage gemäß Anspruch 1 oder 2, **dadurch gekennzeichnet, dass** die Öffnungen der unteren Ebene in der Seitenfläche der Ummantelung ausgeführt sind.

**5.** Anlage gemäß Anspruch 1, **dadurch gekennzeichnet, dass** die UV-Strahlungsquelle waagerecht angebracht ist und die Öffnungen der oberen und unteren Ebenen in der Seitenfläche der Ummantelung ausgeführt sind.

## Revendications

**1.** Dispositif pour la décontamination de l'air qui comprend un corps, dans lequel se trouve le bloc d'alimentation et de commande, comprenant un condensateur de stockage, une source d'alimentation en courant continu haute tension, un générateur d'impulsions d'allumage, un transformateur d'impulsions sur un noyau de ferrite et un bloc de commande à programme, et une source du rayonnement ultraviolet montée sur le corps sous la forme d'une lampe à décharge à impulsion de gaz logée dans un carter tubulaire refroidi, transparent au rayonnement bactéricide, dans lequel le condensateur de stockage et la lampe à décharge à impulsion de gaz forment un circuit de décharge connecté au générateur d'impulsions de démarrage via le transformateur d'impulsions sur un noyau de ferrite, **caractérisé en ce que** la lampe à décharge à impulsions de gaz est installée dans un carter transparent au rayonnement bactéricide avec des capacités de refroidissement par air de convection grâce à la création d'un appel naturel à l'intérieur du carter, dans lequel le carter a dans sa partie supérieure un ou plusieurs orifices de niveau supérieur, et dans sa partie inférieure des orifices de niveau inférieur, avec le rapport suivant de paramètres :

$$h.\ \frac{S^2_{sup.} \cdot S^2_{inf.}}{S^2_{sup.} + S^2_{inf.}} = \frac{1}{A} \cdot C^2 U^4_0 F^2 \qquad (1)$$

où h - distance entre les orifices du niveau supérieur et les orifices du niveau inférieur, m :
$S_{aup.}$ - superficie totale des orifices du niveau supérieur, $m^2$ ;
$S_{inf.}$ - superficie totale des orifices de niveau inférieur, $m^2$ ;
A= $(2 \div 30).10^{13}$ $J^2/m^5s^2$) - coefficient énergétique de corrélation ;
C - capacité du condensateur de stockage d'énergie, F ;
$U_0$ - tension de charge du condensateur de stockage d'énergie, V ;
F - fréquence de répétition des impulsions du générateur d'impulsions d'allumage, Hz ;

**2.** Dispositif selon la revendication 1, **caractérisé en ce que** la source du rayonnement ultraviolet est fixée verticalement sur le corpus.

**3.** Dispositif selon la revendication 2, **caractérisé en ce que** la lampe à décharge est réalisée en forme de U.

**4.** Dispositif selon les revendications 1 ou 2, **caractérisé en ce que** les orifices du niveau inférieur sont réalisés dans la superficie latérale du carter.

**5.** Dispositif selon la revendication 1, **caractérisé en ce que** la source du rayonnement ultraviolet est installée horizontalement, et les orifices des niveaux supérieur et inférieur sont réalisés dans la superficie latérale du carter.

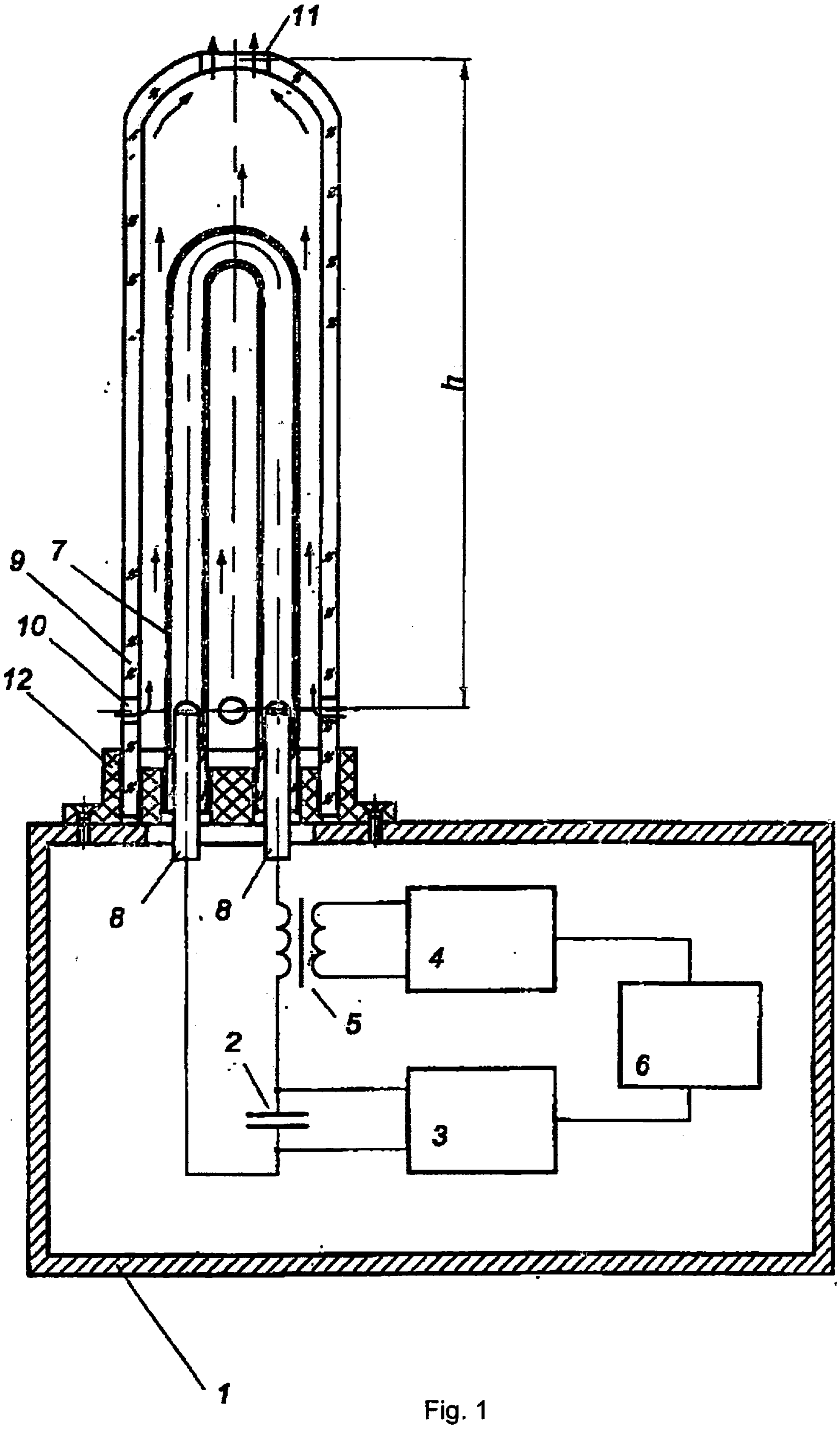


Fig. 1

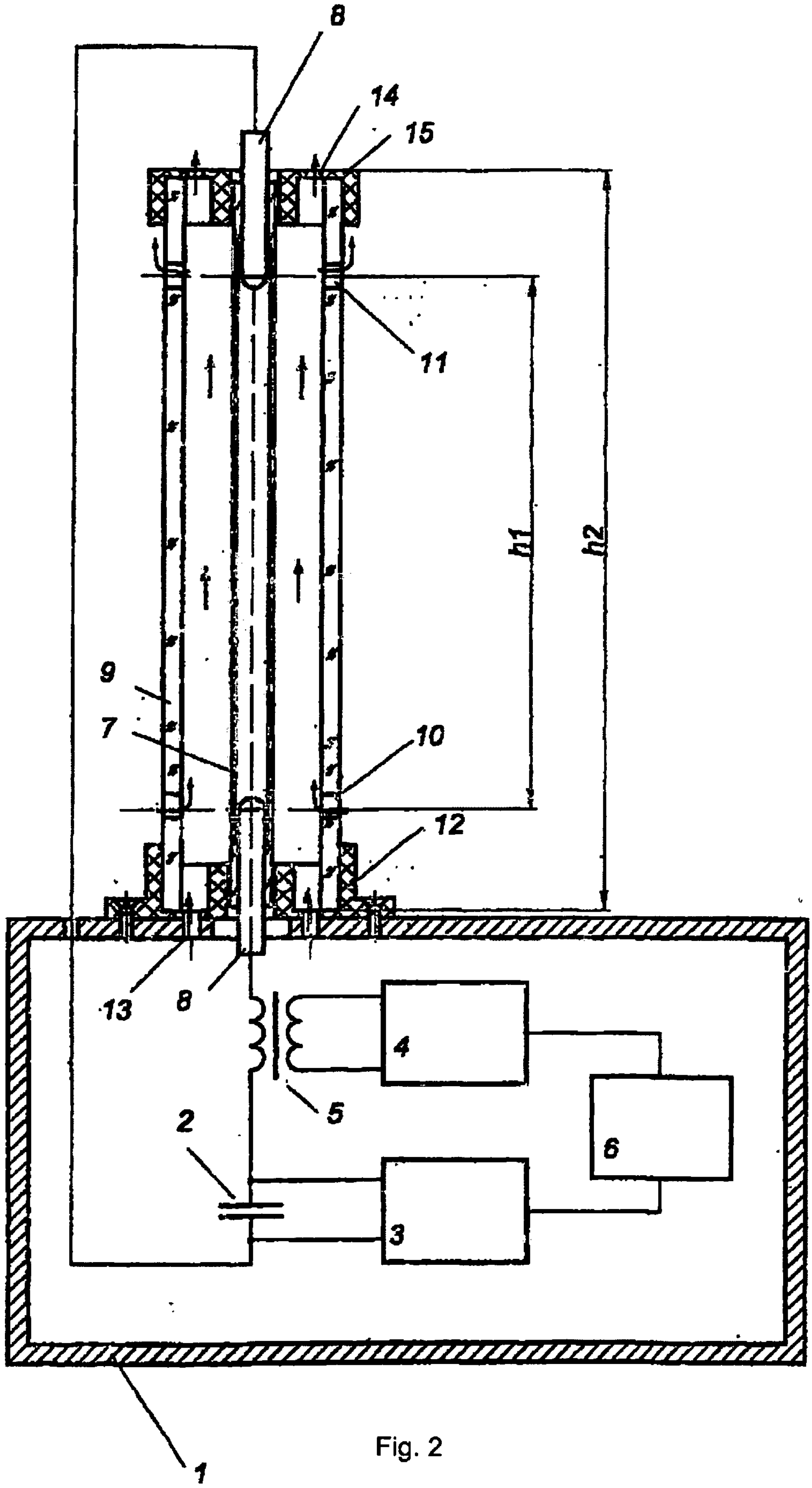


Fig. 2

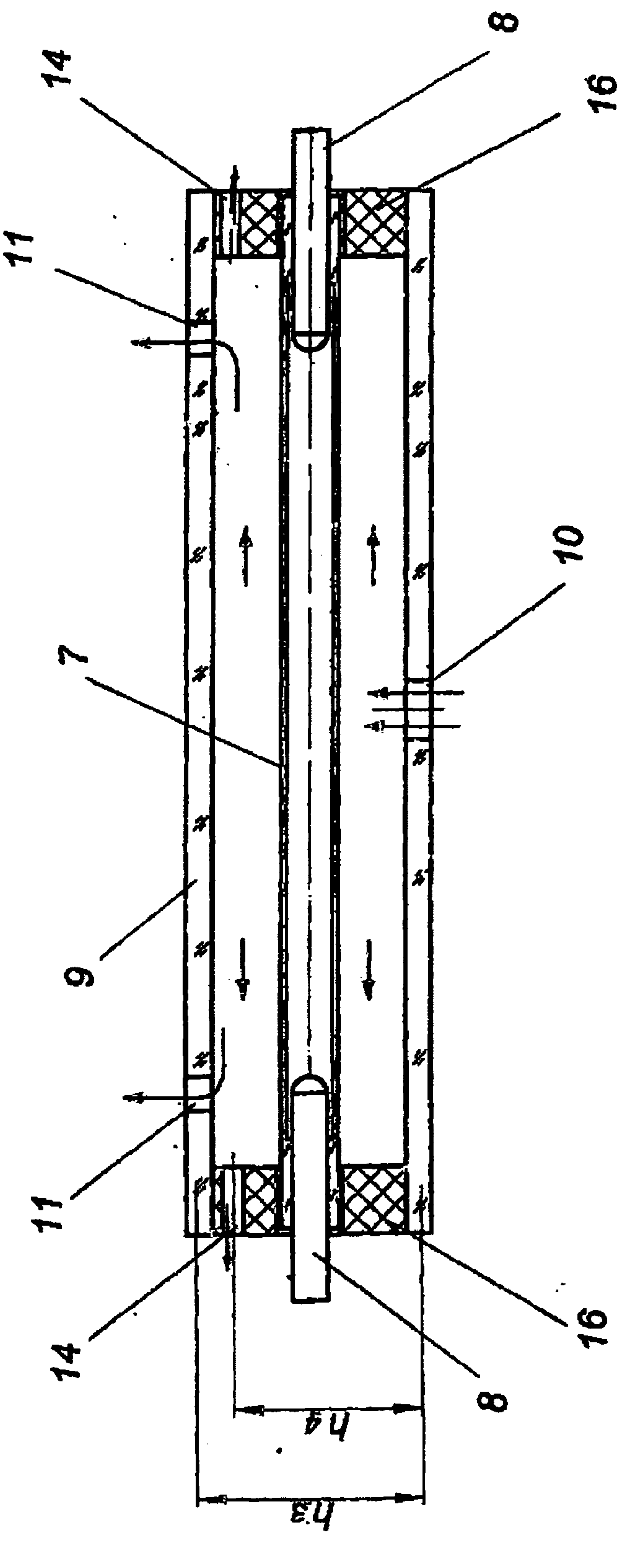


Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- RU 2153886 C1
- EP 0220050 A
- RU 2092191 C1